# EUROPEAN PATENT SPECIFICATION

(11) **EP 2 013 138 B1**
(45) Date of publication and mention of the grant of the patent: **09.04.2014**
(21) Application number: 07746327.1
(22) Date of filing: 03.05.2007
(51) Int. Cl.: A61K 9/51, A61K 9/16

(54) **METHOD FOR PREPARING NANO-SCALE PARTICLE OF ACTIVE MATERIAL**
VERFAHREN ZUR HERSTELLUNG VON TEILCHEN IM NANOMASSSTAB AUS AKTIVEM MATERIAL
PROCÉDÉ DE PRÉPARATION DE NANOPARTICULES DE MATIÈRE ACTIVE

(30) Priority: 04.05.2006 KR 20060040317; 04.05.2006 KR 20060040416
(43) Date of publication of application: 14.01.2009
(73) Proprietor: Bio-Synectics Inc., Seoul 121-854 (KR); Kim, Kab-Sig, Seoul 121-854 (KR)
(72) Inventor: KIM, Kab Sig, Mapo-gu Seoul 121-854 (KR); CHO, Young Tai, Seoul 121-854 (KR)
(74) Representative: Schindele, Claus
(86) International application number: PCT/KR2007/002172
(87) International publication number: WO 2007/129829

(56) References cited:
- WO-A1-2005/054122
- US-B2- 6 966 990
- US-B2- 6 998 051
- US-B2- 7 001 581

## Description

### TECHNICAL FIELD OF THE INVENTION

The present invention relates to a method for preparing nanoscale particles of active material with using a gas of a supercritical fluid, by dissolving an active material into a solvent which is in solid phase at room temperature, to produce nanoscale particles of the active materials which can be advantageously used in medicine, cosmetics, functional foods or the like.

### BACKGROUND OF THE INVENTION

A demand for a technique of an effective and rapid preparation of very fine particles in regular size has been constantly required in various industrial fields. Such fine particles in regular size have many advantages, particularly among which good flowability and little deviation in particle interaction are very advantageous in industrial application. In medical field, the particle size of a therapeutic agent greatly affects to the dissolution rate, bioavailability, formulation and the like, and for example, the smaller the deviation in the interaction between the particles of a therapeutic agent is, the better the whole stability of the therapeutic agent becomes.

When the particle of a therapeutic agent is made into nanoscale size in medicinal products, following advantages may be obtained. First of all, in a drug having a small enteral absorption rate in oral administration, one having a smaller size can be absorbed more than one having a bigger size, thereby increasing the bioavailability of the therapeutic agent. Further, the dosage form of drugs can be varied, for instance a drug being possibly administered only via oral route can be administered by inhalation. In a controlled-release drug formulation, the release rate of a therapeutic agent is a very important factor. When the particle size of the therapeutic agent is formed to be in nanoscale, the particle size becomes relatively more uniform, thus the release rate can become more expectable, thereby being possible to provide more effective therapeutic agent.

In order to take various advantages of regular nanoparticles as described above, many attempts have been made to prepare an active ingredient as a nanoparticle. For this object, mechanical techniques such as crushing, grinding, milling and the like have been conventionally employed to make relatively large particles smaller. In the pharmaceutical industry, a method of milling a mass amount of drugs to the size range being suitable for the medicinal or pharmaceutical use with an air-jet mill has been commonly used. However, such mechanical process involves the risk of contamination and had a limitation on decreasing the particle size to about tens of micrometers.

US patent No. 5,145,684 discloses a method for preparing particles of poorly water-soluble drugs in the size of hundreds of nanometers by wet milling the poorly water-soluble drugs in the presence of a surface modifier. This technique should be applied after a preparation of the drugs in the particle size of not more than 100 micrometer by using a conventional milling process. Generally in this method, the time taken for the preparation of particles having a targeted size range depends on the particular mechanical device used thereto. For example, when using a ball mill, processing times of up to 5 days or longer may be required, however, when using a high shear media mill, 1 day would be enough to provide particles of a desired size. However, in connection with the use of a high shear media mill, contamination associated with the high corrosion of grinding media and grinding vessel should be concerned. Further, a drying process such as spray or freeze drying should be conducted for getting powder form, because the resulted nanoparticles from the wet milling method are in liquid phase. During the drying process, coagulation of the particles is occurred due to interparticle attraction forces, hence it is substantially difficult to obtain a dispersion of particles in a nanometer scale by redispersing the resulted powder into a liquid. In order to solve such problem, US Patent No. 5,302,401 describes an anti-coagulating agent employed during lyophilization. Additionally, US Patent No. 6,592,903 B2 describes an invention comprising a stabilizer, a surfactant and an anti-coagulating agent used during a spray dry process. Further, US Patent No. 2003/0185869 A1 describes an application of a wet milling technique for some poorly soluble drugs, with using lysozyme as a surface stabilizer. However, such protein surface stabilizer used therein has many restrictions in a drying process, accordingly it only describes the preparation in liquid phase.

Other conventionally available methods include a recrystallization technique which provides fine particles of an active ingredient by changing the environment of a solution containing dissolved active ingredient to cause the precipitation or crystallization of solutes. The recrystallization technique can be practiced in two different ways: the one being comprised of dissolving a therapeutic agent in a suitable solvent and lowering the temperature, thereby changing the solubility of the therapeutic agent to precipitate particles; and the other being comprised of adding antisolvent to a solution containing dissolved therapeutic agent, thereby decreasing the solubility of the solute to precipitate particles. However, the recrystallization technique usually requires the use of toxic organic solvent and often causes flocculation or coagulation of the particles during a drying process in wet condition, following after the filtration of the precipitated particles. As a result, the final particles may be irregular in their size.

US Patent No. 2003/0104068 A1 discloses a method for preparing fine particles comprising: dissolving polymers into an organic solvent; dissolving or dispersing a proteineous drug thereto; then rapidly cooling the solution to ultra-low temperature for solidification; and lyophilizing the resulted product to provide a fine powder. In this case, however, there are concerns for the denaturation of a proteineous drug by the contact with an organic solvent and the process economy owing to the rapid cooling and lyophilizing process.

Other techniques for reducing particle size include emulsification. The emulsifying method is commonly used in cosmetic field, which comprises melting poorly water soluble substances with heat or dissolving them in an organic solvent, and then adding the melted or dissolved substances to an aqueous solution containing a surfactant dissolved therein, with stirring at high speed or with sonication to disperse the added substances and provide fine particles. However, in this emulsification method, a step for removing water is required for providing the fine particles in a powdered form, and the step gives variously restrictions to the process. Further, when using an organic solvent to dissolve the poorly water-soluble substance, there always has been a concern for residual toxic organic solvent.

US Patent No. 2004/0067251 A1 discloses a method for preparing fine particles by dissolving active ingredients into an organic solvent and spraying the resulted solution to an aqueous solution containing a surfactant dissolved therein. The invention involves the use of an organic solvent, and requires a drying process for removing the water used, to provide the particles as a powdered form, since the resulted particles are present in aqueous phase. During the drying process, the coagulation of the particles is likely to be occurred, hence the coagulated particles are hardly redispersed with maintaining the particle size to a nanoscale.

Recently, many attempts have been made to use a supercritical fluid in the amorphous or nanoscale particle preparation. Supercritical fluid is a fluid existing in liquid form at a temperature higher than its critical temperature and under pressure higher than its critical pressure. Commonly used supercritical fluid is carbon dioxide. As one of techniques involving the use of supercritical fluids in a nanoparticle preparation, the rapid expansion of a supercritical solution (hereinafter, RESS) is known from the following literatures: Tom et al. Biotechnol. Prog. 7(5):403-411. (1991); US Patent No. 6,316,030 B1; US Patent No. 6,352,737 B1; and US Patent No. 6,368,620 B2. According to RESS, an object solute is firstly dissolved in a supercritical fluid, and then the supercritical solution is rapidly sprayed into a relatively low-pressure condition via nozzle. Then, the density of the supercritical fluid rapidly falls down. As a result, the ability of the supercritical fluid to solubilize the solute is also rapidly reduced, and the solutes are formed into very minute particles or crystallines.

Other techniques using a supercritical fluid include a gas-antisolvent recrystallization (hereinafter, GAS) (Debenedetti et al. J. Control. Release 24:27-44. (1993); WO 00/37169). The method comprises dissolving a therapeutic agent in a conventional organic solvent to prepare a solution and spraying the resulted solution into a supercritical fluid served as an antisolvent, through a nozzle. Then, the volume becomes rapidly expanded upon the contact between the solution and the supercritical fluid. As a result, the density and capacity of the solvent become so much lower to cause excessive supersaturation, hence the solutes form seeds or particles.

US Patent No. 6,630,121 describes a method for preparing fine particles by nebulizing a solution containing active ingredients to provide fine particles with the use of a supercritical fluid, and drying the resulted particles with a dry gas. The method can be used regardless of the solubility of the active ingredients to the supercritical fluid. WO 02/38127 A2 describes a method using SEDS (Solution Enhanced Dispersion by Supercritical fluids) technique for preparing fine particles of active ingredients and coating the resulted fine particles with an additive such as a polymer. Further, US Patent No. 6,596,206 B2 describes a technique of preparing fine particles of active ingredients by dissolving the active ingredients in an organic solvent and focusing acoustic energy to the resulted solution so that the solution can be ejected into a supercritical fluid as a form of fine particles.

Those above-mentioned prior arts propose a method for producing very fine particles with relatively uniform size, but have several disadvantages.

The first disadvantage is likely to occur in a tube for transferring a solution and a nozzle. In a preparation method of fine particles using a supercritical fluid, the particle size generally determined by the diameter of a nozzle used in the method, accordingly the diameter of a nozzle ought to be very fine and precise. However, upon the repeated use of a nozzle, the diameter of the nozzle becomes changed, hence the particle size becomes irregular as time elapses. Moreover, due to the use of a nozzle having an ultra-fine diameter for the preparation of ultra-fine particles, the clogging of the nozzle is likely to occur very often. Further, during unclogging of the nozzle, caking of the particles remained in the tube is frequently occurred.

The second disadvantage of the prior arts is that the species of solutes applicable and solvents available are very limited. The RESS technique can be suitably applied only provided that the solutes are well dissolved in a supercritical fluid. Depending on the solutes, the solubility thereof is possibly increased with the use of a co-solvent, however, if the amount of co-solvent increases, the existence of the residual solvent after the particle generation would cause the growth of crystals, which obstructs the preparation of the particles in regular size. In the GAS technique, a solvent should be selected with great concern. Only provided that the solvent containing the solutes dissolved therein is rapidly diffused into the supercritical fluid as being contacted together, fine particles can be generated. Further, the growth of particles can be prevented, provided that the amount of solvent remained between the particles during filtration is minimized. In addition, the GAS technique requires a special filtration device for filtering the resulted fine particles from the solvent.

The third disadvantage of the prior arts is that there are many restrictions in commercial scale production of nanoparticles by those conventional methods using a supercritical fluid. For the commercial scale use of RESS, solutes used should be very soluble in a supercritical fluid, which are very rare. Further, the preparation of nanoscale fine particles of a single species of material involves the coagulation of the particles, hence an anti-coagulating material such as an emulsifier, cellulose or lipids should be dissolved together, and the mixture thereof should be made into fine particles in nanoscale. However, most of the anti-coagulating materials would not be soluble in carbon dioxide which is mainly used as a supercritical fluid. In preparing nanoparticles using GAS, the solution containing solutes dissolved therein is injected into a reaction vessel containing a supercritical fluid, but the injection rate is so slow that the preparation of uniform-sized particles is difficult. However, when increasing the injection rate, the particle sizes become irregular and further problems would be occurred in a filtration process. Moreover, the resulted particles with the composition ratio, which was not originally intended, would be obtained instead of particles with desired composition ratio, due to the differences between the solubility of the solutes to the solvent and the solubility of the anti-coagulating material added thereto, for preventing the coagulation of particles.

Korean patent application No. 2004-90832 and WO 2005/054122 describe a method for preparing nanoscale particles by using a fat maintaining solid phase at 30 °C or less, as a solvent, such as saturated fatty acids, esters and alcohols with C10~C22, and using a gas of a supercritical fluid at the temperature of 20-40 °C under the pressure of 70~200atm).

### SUMMARY OF THE INVENTION

The object of the present invention is to provide a method for preparing nanoscale particles of active ingredients by using a subcritical or supercritical fluid at low temperature under low pressure, to prepare nanoscale particles with good efficiency.

### DETAILED DESCRIPTION OF THE INVENTION

According to the present invention, provided is a method for preparing nanoscale particles of active ingredient, comprising the steps of: (1) preparing a mixture comprising one or more active ingredients and solid solvent, (2) pressurizing the mixture comprising one or more active ingredients and solid solvent to 41 to 91 bar (40 to 90 atmosphere), at 10 to 20 °C by adding the gas of a supercritical fluid into a reaction vessel containing the mixture, and (3) removing the solid solvent from the mixture by releasing out the solid solvent together with the gas of the supercritical fluid, with maintaining the temperature and pressure in the reaction vessel at 10 to 20 °C, and 41 to 91 bar (40 to 90 atmosphere), respectively.

The term "gas of a supercritical fluid" used herein, refers to an inert gas, which has no reactivity such as a carbon dioxide gas or a nitrogen gas, but can be a supercritical fluid under specific temperature and pressure conditions, i.e. beyond their critical point.

The terms, "critical temperature" and "critical pressure" used herein, refer to specific temperature and pressure, respectively, under which the gas of a supercritical fluid can be liquefied as a supercritical fluid.

The terms, "subcritical temperature" and "subcritical pressure" used herein, refer to temperature and pressure around the critical temperature and pressure, respectively. For example, in case that the gas of a supercritical fluid is carbon dioxide gas, the subcritical temperature and pressure conditions may mean, but not limited to, a temperature condition of 32 °C or less and a pressure condition of 71 bar (70 atmosphere) or less, respectively.

The active ingredients useful in the method for preparing nanoscale or amorphous particles (hereinafter, referred as "nanoparticles") according to the present invention include, for example, organic compounds, organometallic compounds, natural extracts, peptides, proteins, polysaccharides or the like, which exhibit specific physiological activities in medicinal products, functional foods, cosmetics or the like, and there is no specific restriction on their phase at room temperature such as solid or liquid phase and electrical properties such as being neutral or ionic.

The term, "nanoparticles" used herein, refers to particles wherein 90% or more of the particles have a size of 5µm or less, preferably 2µm or less, more preferably 1µm or less, still more preferably 0.5µm or less.

The solid solvent, which may also be referred as "solid fat," useful in the method for preparing nanoparticles according to the present invention, is a compound or a mixture thereof maintaining solid phase at room temperature, i.e. at 30 °C or less, having a relatively low melting point as being 30 to 150°C, preferably 30 to 90°C and showing a large solubility in supercritical fluid. For example, the solid solvent used in Korean patent application No. 2004-90832 may also be used in the present invention. For instance, the solid solvent may be one or more selected from the group consisting of saturated fatty acids, esters and alcohols with C10~C22; mono- or di-glycerides having saturated fatty acid group with C10~C22; hydrocarbons with C16 or more; compounds having reduced fatty acid of tri-glycerides with C10~C22; linear or branched diol compounds with C6-C22, preferably C6~C10 such as 1,6-hexanediol; and mixtures thereof.

According to one preferred embodiment of the present invention, when a mixture of diol compound and other solid solvent than diol is used as the solid solvent, the amount of the other solid solvent used is preferably, but not limited to, 1~1000 parts by weight per 100 parts by weight of the diol compound used, in terms of production efficiency of nanoparticles.

In the method for preparing nanoparticles of the present invention, the mixture comprising one or more active ingredients and solid solvent in the step (1) may further comprise a surfactant. Preferably, the surfactant may be one or more selected from the group consisting of synthetic surfactants, natural surfactants, lipids and polymers.

Also, in the method for preparing nanoparticles of the present invention, the mixture comprising one or more active ingredients and solid solvent in the step (1) may further comprise a non-surfactant type anti-coagulating (or anti-aggregating or anti-crystallizing) agent. Preferably, the non-surfactant type anti-coagulating agent may be one or more selected from the group consisting of monosaccharides, polysaccharides, dietary fibers, gums and proteins.

In the method for preparing nanoparticles of the present invention, the nanoparticles may be prepared by using the active ingredients as a single component. Optionally, an anti-coagulating agent may be further used for preventing the coagulation of the resulted nanoparticles. Such anti-coagulating agents useful in the present invention may be classified into a surfactant type and a non-surfactant type. As the surfactant type anti-coagulating agent, various synthetic and natural surfactants, lipids, polymers and the like may be used. As the non-surfactant type anti-coagulating agent, monosaccharides, polysaccharides, dietary fibers, gums, proteins and the like may be used. Phospholipids such as lecithin, lysolecithin, phosphatidyl choline, phosphatidyl ethylamine and the like are referred herein as a surfactant, though it may be classified as lipids in general. Surfactants may be generally divided, upon their affinity to water, into a hydrophilic type and a lipophilic type, which are determined by the HLB (hydrophilic-lipophilic balance) value. Upon the functional groups, there are four types of surfactants such as cationic, anionic, neutral and zwitterionic. A surfactant or non-surfactant type anti-coagulating agent useful in the present invention is not specifically restricted to a certain type or species, as long as it prevents the coagulation of the active ingredients, and it is well dissolved in the solid solvent and is not readily removed by a supercritical fluid.

According to one preferred embodiment of the present invention, when diol compound is used as the solid solvent, some materials showing low solubility in a general solid solvent other than diol, for example, polymeric surfactant or anti-coagulating agent such as Eudragit or hydroxypropyl methyl cellulose, may be dissolved well. Thus, such surfactant or anti-coagulating agent can be utilized to prepare nanoparticles of some active ingredients which are hard to prepare in nanoscale particles by using general solid fat. In case that active ingredients can be prepared in nanoscale particles by using general solid fat, the production efficiency can be improved. Also, if active ingredient is sensitive to heat, the activity loss of the active ingredient due to heat during the production of nanoparticles can be reduced since diol compound can dissolve active ingredient or the like relatively low temperature, compared with general solid fat.

Further, when sufficient dissolution of the active ingredients and surfactants is not achieved by using only solid solvent, one or more co-solvents selected from the group consisting of alcohol, water and mixtures thereof may be further used in the method of the present invention. For the alcohol as the co-solvent, lower alcohol with C2-C6 is preferred, and ethanol is the most preferred. When a mixture solution of alcohol and water is used as the co-solvent, a mixture solution of 70-80 wt% of alcohol and 20-30 wt% of water is preferred. Also, active ingredients may be dissolved together with anti-coagulating agent such as sucrose, lactose and xylitol, by using the co-solvent such as alcohol or water.

In the step (2) of the method for preparing nanoparticles according to the present invention, the mixture comprising one or more active ingredients and solid solvent is pressurized to 41 to 91 bar (40 to 90 atmosphere), at 10 to 20 °C, by adding the gas of a supercritical fluid into a reaction vessel containing the mixture.

In the step (3) of the method for preparing nanoparticles according to the present invention, the solid solvent is removed from the mixture by releasing out the solid solvent together with the gas of the supercritical fluid, with maintaining the temperature and pressure in the reaction vessel at 10 to 20 °C and 41 to 91 bar (40 to 90 atmosphere), respectively.

In the steps (2) and (3) of the method for preparing nanoparticles according to the present invention, when the temperature condition is less than 10 °C or the pressure condition is less than 41 bar (40 atmosphere), the productivity of whole process becomes worse since the solid solvent is not removed easily. To the contrary, when the temperature condition is greater than 40 °C or the pressure condition is greater than 405 bar (400 atmosphere), the loss of active ingredients may happen.

In the steps (2) and (3) of the method for preparing nanoparticles according to the present invention, the temperature and pressure conditions may be selected appropriately, according to the specific kinds of active ingredient or solid solvent, or in order to improve the efficiency of nanoparticle production.

For example, in case that a low temperature - low pressure condition is needed to improve the efficiency of nanoparticle production, the temperature and pressure in the steps (2) and (3) of the present method is set to, respectively, 10 to 20 °C and 41 to 91 bar (40 to 90 atmosphere), preferably 51 to 81 bar (50 to 80 atmosphere), more preferably 51 to 71 bar (50 to 70 atmosphere). Such low temperature - low pressure condition is particularly useful in case that the active ingredients are released out together with the gas of the supercritical fluid or the solid solvent in which the active ingredients are dissolved, and so the resulting particle size distribution becomes broad and the average particle size becomes too large. Also, when the active ingredients are sensitive to heat, the low temperature - low pressure condition can reduce the loss of activity due to the heat during the procedure for producing nanoparticles. Further, since the solid solvent is removed under lower pressure condition than conventional ones, high-pressure equipments are not required and thus the costs for the equipments and operation can be reduced.

Hereinafter, the method for preparing nanoparticles of the present invention is now illustrated step by step with more details.

In the step (1) of the method for preparing nanoparticles according to the present invention, a mixture comprising one or more active ingredients and solid solvent is prepared. The details thereof are now described as follows.

According to one preferred embodiment of the present invention, the step (1) comprises: adding one or more active ingredients, solid solvent and optionally one or more surfactants into a reaction vessel and melt-mixing them homogeneously.

According to other preferred embodiment of the present invention, the step (1) comprises: adding one or more active ingredients, solid solvent and optionally one or more surfactants into a reaction vessel and melt-mixing them homogeneously; rapidly cooling the mixture for solidification; pulverizing the solidified mixture; adding one or more surfactants and/or one or more non-surfactant type anti-coagulating agents or aqueous solution thereof to the pulverized powder and mixing them homogeneously; and drying the mixed product at room temperature.

According to another preferred embodiment of the present invention, the step (1) comprises: adding one or more surfactants and solid solvent into a reaction vessel, and melt-mixing them homogeneously; rapidly cooling the mixture for solidification; pulverizing the solidified mixture; adding one or more surfactants and/or one or more non-surfactant type anti-coagulating agents together with one or more active ingredients or aqueous solution thereof, to the pulverized powder and mixing them homogeneously; and drying the mixed product at room temperature.

According to another preferred embodiment of the present invention, the step (1) comprises: adding one or more active ingredients, solid solvent and optionally one or more surfactants into a reaction vessel, further adding the gas of a supercritical fluid, and then melt-mixing the mixture by heating.

According to another preferred embodiment of the present invention, the step (1) comprises: adding one or more active ingredients, solid solvent and optionally one or more surfactants into a reaction vessel, pressurizing the mixture by adding the gas of a supercritical fluid into the mixture and then melt-mixing the mixture, and spraying the melted mixture to the atmospheric pressure.

According to another preferred embodiment of the present invention, the step (1) comprises: adding one or more active ingredients, solid solvent and optionally one or more surfactants into a reaction vessel, pressurizing the mixture by adding the gas of a supercritical fluid and then melt-mixing the mixture, and pulverizing the melted mixture by spraying it to the atmospheric pressure; adding one or more surfactants and/or one or more non-surfactant type anti-coagulating agents or aqueous solution thereof to the pulverized mixture and mixing them homogeneously; and drying the mixture at room temperature.

According to another preferred embodiment of the present invention, the step (1) comprises: adding one or more active ingredients, solid solvent and optionally one or more surfactants into a reaction vessel, and melt-mixing the mixture homogeneously; adding one or more surfactants and/or one or more non-surfactant type anti-coagulating agents or aqueous solution thereof to the melted mixture and mixing them homogeneously; rapidly cooling the mixture for solidification; and pulverizing and drying the solidified mixture.

According to another preferred embodiment of the present invention, the step (1) comprises: adding solid solvent and optionally one or more surfactants into a reaction vessel, and melt-mixing the mixture homogeneously; adding one or more active ingredients and one or more surfactants and/or one or more non-surfactant type anti-coagulating agents or aqueous solution thereof to the melted mixture and mixing them homogeneously; rapidly cooling the mixture for solidification; and pulverizing and drying the solidified mixture.

According to one preferred embodiment of the present invention, one or more active ingredients and solid fat are added into a reaction vessel wherein the amount of the solid solvent is 0.1~1000 parts by weight per 1 part by weight of the active ingredients. At this stage, when necessary, 0.001~10 parts by weight of surfactant, or 0.001~10 parts by weight of lower alcohol or a mixture solution of 70 to 80 wt% of the alcohol and 20 to 30 wt% of water as co-solvent, or a mixture of 0.001~10 parts by weight of surfactant and 0.001~10 parts by weight of lower alcohol or a mixture solution of 70 to 80 wt% of the alcohol and 20 to 30 wt% of water as co-solvent, based on 1 part by weight of the active ingredients may be optionally added to the reaction vessel. Also, preferably, 0.01-50 parts by weight of an aqueous solution of one or more non-surfactant type anti-coagulating agents selected from the group consisting of monosaccharides, polysaccharides, dietary fibers, gums and proteins may be added, based on 100 parts by weight of the solid solvent.

The optionally added surfactant should have relatively large solubility to the solid solvent so as to form a homogeneous solution when being dissolved together with the active ingredients in solid solvent, or in solid solvent containing a lower alcohol described above. Further, different surfactants may be selected, depending on the properties of the active ingredients and the use or the purpose of use of the resulted nanoparticles. When the resulted nanoparticles are used finally in the form of a water dispersion, a surfactant with a high HLB value is preferably selected, and when the purpose is to increase the internal absorption rate, a surfactant with a relatively low HLB value is preferably selected.

As mentioned above, the active ingredients and solid solvent are added to a reaction vessel and when being necessary, surfactant or lower alcohol or a mixture solution of 70 to 80 wt% of the alcohol and 20 to 30 wt% of water as co-solvent, is further added to the reaction vessel, and then the mixture in the reaction vessel is gradually melted as being heated. If necessary, the surfactant and co-solvent may be added after the active ingredients and solid solvent are melted clearly.

As the temperature inside the reaction vessel rises, the solid solvent becomes melt, and the active ingredients and surfactant or the like are dissolved therein. The temperature is raised until a homogeneous solution is formed. It is preferred to start stirring from the point when it becomes possible, since it will make the solution of the mixture more homogeneous and reduce the working time. The point when stirring becomes possible depends on the specific kinds of the active ingredients, surfactant and solid solvent used in the method, however the determination of the starting point of stirring will be easily made at the working site by the skilled person in this field.

According to other preferred embodiment of the present invention, as it has been mentioned above, a mixture comprising one or more active ingredients and solid solvent is prepared by: adding the one or more active ingredients, solid solvent and optionally one or more surfactants to a reaction vessel; melt-mixing them together homogeneously; rapidly cooling the resulted mixture for solidification; pulverizing the solidified mixture; adding one or more surfactants and/or one or more non-surfactant type anti-coagulating agents or aqueous solution thereof to the resulted powder, and mixing them homogeneously; and drying the resulted mixture at room temperature. In the above processes, the drying process is not particularly restricted to a certain method, but it should be conducted below the melting point of the solid solvent used. The term, "melting point of the solid solvent" used herein, refers to the temperature at which the melting of the surface of the solid solvent is observed first as the temperature rises.

According to another preferred embodiment of the present invention, when the active ingredients are those sensitive to the temperature or soluble in water such as peptides, proteins or polysaccharides, the mixture comprising the active agents and solid solvent is prepared by: firstly, adding one or more surfactants and solid solvent into a reaction vessel and melt-mixing them homogeneously; rapidly cooling the melted mixture for solidification; pulverizing the solidified mixture; then adding the active ingredients together with one or more surfactants and/or one or more non-surfactant type anti-coagulating agents or aqueous solution thereof, to the resulted powder, and mixing them homogeneously; and drying the resulted mixture at room temperature. In the above processes, the drying process is not particularly restricted to a certain method, but it should be conducted below the melting point of the solid solvent used.

In the solidification of the mixture by rapid cooling, it is preferred to rapidly decrease the temperature of the solution of the melted mixture to the temperature of 10°C or less. When cooling is conducted slowly, crystal growth of the active ingredients may occur, and under such circumstances, the nanoparticles of the active ingredients are hardly achieved and the obtained particles are likely to have a broad particle distribution.

The solid product obtained from the rapid cooling, is conventionally milled by, for example, dry milling and the like. The smaller the size of the milled particles is, i.e. the larger the surface area of the particles is, the more it is advantageous in later processes such as a fat removal process. The particle size after the milling process is preferably 100 micrometer or less, but not limited thereto.

According to another preferred embodiment of the present invention, the mixture comprising one or more active ingredients and solid solvent is prepared by: adding the one or more active ingredients, solid solvent and optionally one or more surfactants to a reaction vessel; further adding the gas of a supercritical fluid (for instance, CO₂ gas) to the mixture, preferably so as to form subcritical or supercritical conditions; and then melting the resulted mixture by heating.

According to another preferred embodiment of the present invention, the mixture comprising one or more active ingredients and solid solvent is prepared by: adding the one or more active ingredients, solid solvent and optionally one or more surfactants to a reaction vessel; adding thereto the gas of a supercritical fluid, preferably up to the pressure over the critical pressure and melting the mixture; and then spraying the melted mixture to the atmospheric pressure.

According to another preferred embodiment of the present invention, a mixture comprising one or more active ingredients and solid solvent is prepared by: adding the one or more active ingredients, solid fat and optionally one or more surfactants to a reaction vessel; adding thereto the gas of a supercritical fluid, preferably up to the pressure over the critical pressure and melting the mixture; then spraying the melted mixture to the atmospheric pressure for pulverization; adding one or more surfactants and/or one or more non-surfactant type anti-coagulating agents or aqueous solution thereof to the resulted mixture and mixing homogeneously; and drying the mixture at room temperature. In the above processes, the drying process is not particularly restricted to a certain method, but it should be conducted below the melting point of the solid solvent used.

In the case of using a supercritical fluid in the step (1) of the present invention, after the components of the mixture are completely melted and homogeneously mixed, a supercritical fluid such as CO₂ is slowly added into a reaction vessel to pressurize the mixture, preferably up to the pressure under which the gas of a supercritical fluid is liquefied as a supercritical fluid, i.e. the critical pressure (for CO₂, 70 atm) or more. The pressure inside the reaction vessel at this stage depends on the reaction vessel size and the amount of the mixture. The temperature at this stage is a temperature that can provide the sufficient fluidity to the solution of the mixture for stirring.

Once the critical pressure or more is achieved by raising the pressure inside the reaction vessel with the gas of a supercritical fluid, it is preferred to carry out stirring for additional 10 minutes or more at that condition, so that the supercritical fluid may be sufficiently permeated into the solution of the mixture.

In completing the additional stirring, while slowly adding thereto the gas of the supercritical fluid further, the exhaust port, which is connected to another reaction vessel under atmospheric pressure, is opened to the full for spraying the resulted solution of the mixture into the reaction vessel under atmospheric pressure. At this moment, the supercritical fluid is instantly vaporized, thereby rapidly cooling down the surroundings and causing the solidification of the resulted solution of the mixture in an instant. The solidification of the solution of the mixture is so instantaneous that it becomes short of energy and time demanded for crystal growth, therefore it is possible to obtain solid products in which the solutes including the active ingredients, surfactant and the like and the solid solvent are homogeneously mixed in the form of very fine particles. In the solid products obtained therefrom, the very fine nanoscale particles of the active ingredients are dispersed uniformly. Further, since the surfactant is also uniformly mixed with the active ingredients, the dispersability and stability of the finally produced fine particles become significantly improved.

The purpose of this step is to make the particles of active ingredients be finer and more uniform in the solid product. Therefore, as long as the particle size of the solid product containing the active ingredients is in the range that does not cause any problem to the workability in subsequent processes, it is not necessary to specifically adjust the particle size of the solid product itself. Accordingly, it is not necessary to adjust the spray nozzle diameter or the spraying rate, in order to adjust the particle size of the solid product itself produced by spraying into the atmospheric pressure condition. Therefore, the risk of deformation or clogging of the spray nozzle does not need to be concerned any more.

In spraying the solution of the mixture into another reaction vessel under the atmospheric pressure condition, a conical supporting plate is preferably placed inside the reaction vessel under the atmospheric pressure condition, at a distance from the spray outlet such as nozzle, in order to solidify the sprayed solution into the form of finer powders. By doing so, the solids can be formed into finer particles, and in the next step, the solid solvent can be more easily removed with the supercritical fluid.

According to another preferred embodiment of the present invention, a homogeneous mixture comprising active ingredient and other additives can be obtained by cooling and pulverizing the melted mixture of active ingredient, surfactant and solid solvent, if necessary, after further adding surfactant and/or non-surfactant type anti-coagulating agent or aqueous solution thereof to the mixture.

According to the preferred embodiment of the present invention, to the powdered mixture obtained by using a supercritical fluid or milling, when being necessary, one or more surfactants and/or one or more non-surfactant type anti-coagulating agents or aqueous solution thereof can be added, or alternatively when the active ingredients are those temperature sensitive or water soluble such as peptides, proteins or polysaccharides, the surfactant and/or the non-surfactant type anti-coagulating agent together with the active ingredients or aqueous solution thereof can be added. The resulted mixture may be homogeneously mixed by using a general mixer.

In the above, when necessary, the non-surfactant type anti-coagulating agent is added in the amount of 0.001~10 parts by weight per 1 part by weight of the active ingredients. When the aqueous solution of surfactant or the non-surfactant type anti-coagulating agent is added, the physical state of the resulted mixture may be varied upon the amount of water used and the kinds of the surfactant and anti-coagulating agent, but if the amount of water added is generally 30%(w/w) or less of the amount of solid solvent used, the mixture will be readily formed into a powder. The amount of water added is not specifically limited, as long as it can sufficiently disperse the water-soluble components into the mixture prepared. When 40%(w/w) or more of water is used, the mixture becomes the form of dough or paste, which can be dried easily at room temperature by various conventional methods. The drying process is not particularly restricted to a certain method, but it should be conducted below the melting point of the solid solvent used. Further, it would be readily understood by the skilled person in this field that, the smaller the particle size used is, the more water can be easily removed by a conventional drying process under reduced pressure. After completing the drying process, the residual water content relative to the solid solvent content is preferably not more than 30%.

The details of the steps (2) and (3) in the present method for preparing nanoparticles are described as follows.

While maintaining the temperature of the reaction vessel containing the mixture obtained from the preceding steps including the step (1) in the range of 10~20°C, the gas of a supercritical fluid is added to the reaction vessel to pressurize it to 41-91 bar, preferably 51-91 bar (40~90 atm, preferably 50~90 atm.). Then, maintaining the reaction vessel under said temperature and pressure by controlling an input valve and an output valve for the gas of a supercritical fluid such as carbon dioxide, the gas of a supercritical fluid is gradually released out. Along with the release of the gas of the supercritical fluid, the solid solvent is also released out, i.e. removed from the reaction vessel. At this stage, by maintaining said temperature and pressure conditions, dissolution and releasing out of the active ingredient together with the supercritical fluid and the solid solvent can be prevented, and the growth of particles of the active ingredient caused by melting and recrystallization of the active ingredient, can be suppressed.

When the supercritical fluid and the solid solvent are removed, the pressure of inside of the reaction vessel is preferably maintained in a range wherein the solid solvent is readily dissolved in the supercritical fluid but the active ingredient is hardly dissolved in the supercritical fluid or solid solvent which is dissolved in the supercritical fluid. Most active ingredients are not dissolved in the supercritical fluid, but some may be dissolved and in such case, the pressure of inside of the reaction vessel during the removal of the supercritical fluid and the solid solvent is preferably maintained to about 50 atm to prevent the dissolution and releasing out of the active ingredient together with the supercritical fluid and the solid solvent.

The time taken for removing the solid solvent with a supercritical fluid is quite dependent on the kinds and amount of the solid solvent used. In order to obtain the particles of active ingredients with higher purity, it is preferred to take time in removing the solid solvent as long as possible, thereby minimizing the residual amount of the solid solvent. The solid solvent preferably used in the present invention is non-toxic to a human body, therefore the residual amount is not particularly limited to a specific range. However, considering the purity of the resulted active ingredients, the residual amount is preferably not more than 10 wt%, preferably not more than 5 wt% of the total weight.

The solid solvent removed from the mixture by the method described above, can be collected in a separate reaction vessel and then used again in future.

Hereinafter, the present invention is illustrated in detail with a reference to the examples as follows, however the present invention is by no means limited to those examples.

### Example 1

30g of myristyl alcohol as a solid solvent was placed into a 250ml volume beaker and slowly heated to 100°C, and then 1 g of polyvinylpyrrolidone (K 30) as a surfactant and 1 g of paclitaxel as an active ingredient were added thereto. The resulting mixture was melted completely and then cooled slowly at room temperature to obtain solid product.

5 g of the resulted solid product was charged into a pressure-resistant reaction vessel. Then, while maintaining the temperature inside the reaction vessel in 15~20°C, a carbon dioxide gas was added to elevate the pressure inside the reaction vessel to 61-91 bar (60~90 atm.). While maintaining said temperature and pressure, myristyl alcohol was removed by continuously adding the carbon dioxide gas for 8 hours. As a result, 0.31 g of mixed powder of paclitaxel and polyvinylpyrrolidone was obtained.

The obtained mixed powder was dispersed into distilled water, and the particle size thereof was determined with using a particle size analyzer (Horiba LA910S), and the result was shown in Table 1.

### Example 2 (comparative)

10 g of the solid product obtained in Example 1 was charged into the pressure-resistant reaction vessel. Then, while maintaining the temperature inside the reaction vessel in 25~32 °C, a carbon dioxide gas was added to elevate the pressure inside the reaction vessel to about 101 bar (100 atm). While maintaining said temperature and pressure, myristyl alcohol was removed by continuously adding the carbon dioxide gas for 10 hours. As a result, 0.62 g of mixed powder of paclitaxel and polyvinylpyrrolidone was obtained.

The obtained mixed powder was dispersed into distilled water, and the particle size thereof was determined with using a particle size analyzer (Horiba LA910S), and the result was shown in Table 1.

**[Table 1]**

| Particle size distribution (*µ*m) of the final powders obtained from Examples 1 and 2 | | | | |
|---|---|---|---|---|
| | D50 | D70 | D90 | Average |
| Example 1 | 0.0930 | 0.1098 | 0.1602 | 0.1073 |
| Example 2 | 0.4954 | 10.1310 | 30.2567 | 8.7291 |

From the result shown in Table 1, it can be known that under high temperature and high pressure, some active ingredients are melted into the solid solvent which is dissolved in the supercritical fluid, and recrystallized, and thus the particle size distribution becomes broad.

### Example 3 (not according to the invention)

21g of cetyl alcohol as a solid solvent was placed into a 250ml volume beaker and slowly heated to 100°C, and then 0.56 g of polyvinylpyrrolidone (K 30) as a surfactant and 0.7 g of itraconazole as an active ingredient were added thereto. The resulting mixture was melted completely and then cooled slowly to 70 °C. 3.85 ml of a solution, which was prepared by dissolving 400 mg of hydroxypropyl methyl cellulose in a mixed solvent of 80% of ethanol and 20% of water, was added thereto and the mixture was stirred sufficiently for 5 minutes and solidified slowly at room temperature to obtain solid product. The obtained solid product was dried for 24 hours at room temperature under reduced pressure.

1.6 g of the resulted solid product was charged into the pressure-resistant reaction vessel. Then, while maintaining the temperature inside the reaction vessel in 22~28°C, a carbon dioxide gas was added to elevate the pressure inside the reaction vessel to 61-91 bar (60~90 atm.). While maintaining said temperature and pressure, cetyl alcohol was removed by continuously adding the carbon dioxide gas for 8 hours. As a result, 0.1 g of mixed powder of itraconazole, hydroxypropyl methyl cellulose and polyvinylpyrrolidone was obtained.

The obtained mixed powder was dispersed into distilled water, and the particle size thereof was determined with using a particle size analyzer (Horiba LA910S), and the result was shown in Table 2.

**[Table 2]**

| Particle size distribution (*µ*m) of the final powders obtained from Example 3 | | | | |
|---|---|---|---|---|
| | D50 | D70 | D90 | Average |
| Example 3 | 0.4640 | 0.5834 | 0.8938 | 0.8499 |

### Example 4 (not according to the invention)

3g of 1,6-hexanediol was placed into a container and slowly heated to 100°C, and then 50 mg of hydroxypropyl methyl cellulose as a surfactant was added thereto. The resulting mixture was melted completely and then cooled to 80°C. 100 mg of itraconazole as an active ingredient was added and stirred to melt it completely to form a transparent liquid. Then, 0.4 g of aqueous solution of lactose (1g/3ml) was slowly added dropwise and the mixture was stirred sufficiently for 5 minutes. Then, the melted mixture was poured into a stainless steel plate at room temperature for solidifying. The obtained solid mixture was dried under reduced pressure to give a solid product wherein fine particles of the active ingredient were dispersed uniformly in solid phase diol.

1.66 g of the resulted solid product was charged into the pressure-resistant reaction vessel. Then, while maintaining the temperature inside the reaction vessel in 18~27°C, a carbon dioxide gas was added to elevate the pressure inside the reaction vessel to 71-101 bar (70~100 atm.). While maintaining said temperature and pressure, 1,6-hexanediol was removed by continuously adding the carbon dioxide gas for 8 hours. As a result, 0.12 g of mixed powder of itraconazole and hydroxypropyl methyl cellulose was obtained.

The obtained mixed powder was dispersed into distilled water, and the particle size thereof was determined with using a particle size analyzer (Horiba LA910S), and the result was shown in Table 3.

### Example 5 (not according to the invention)

3g of 1,6-hexanediol was placed into a container and slowly heated to 100°C, and then 100 mg of polyvinylpyrrolidone (K 30) as a surfactant was added thereto. The resulting mixture was melted completely and then cooled to 80 °C. 100 mg of itraconazole as an active ingredient was added and stirred to melt it completely to form a transparent liquid. Then, 0.4 g of aqueous solution of lactose (1g/3ml) was slowly added dropwise and the mixture was stirred sufficiently for 5 minutes. Then, the melted mixture was poured into a stainless steel plate at room temperature for solidifying. The obtained solid mixture was dried under reduced pressure to give a solid product wherein fine particles of the active ingredient were dispersed uniformly in solid phase diol.

1.7 g of the resulted solid product was charged into the pressure-resistant reaction vessel. Then, while maintaining the temperature inside the reaction vessel in 18~27°C, a carbon dioxide gas was added to elevate the pressure inside the reaction vessel to 71-101 bar (70~100 atm.). While maintaining said temperature and pressure, 1,6-hexanediol was removed by continuously adding the carbon dioxide gas for 8 hours. As a result, 0.14 g of mixed powder of itraconazole and polyvinylpyrrolidone was obtained.

The obtained mixed powder was dispersed into distilled water, and the particle size thereof was determined with using a particle size analyzer (Horiba LA910S), and the result was shown in Table 3.

**[Table 3]**

| Particle size distribution (*µ*m) of the final powders obtained from Examples 4 and 5 | | | | |
|---|---|---|---|---|
| | D50 | D70 | D90 | Average |
| Example 4 | 0.6136 | 0.9971 | 5.7745 | 1.7563 |
| Example 5 | 0.5765 | 0.9014 | 6.0685 | 1.7549 |

### Example 6 (not according to the invention)

0.1 g of mixed powder of itraconazole and Eudragit L-100 was obtained by the same process as in Example 4 excepting that 100 mg of Eudragit L-100 was used as a surfactant.

Eudragit L-100 in the obtained mixed powder was not dissolved in neutral solvent such as distilled water, and so the particle size of the obtained mixed powder could not be determined. However, the state of the final mixed powder was very similar to those of Example 4.

### Example 7 (not according to the invention)

0.1 g of mixed powder of itraconazole and Eudragit S-100 was obtained by the same process as in Example 4 excepting that 100 mg of Eudragit S-100 was used as a surfactant.

Eudragit S-100 in the obtained mixed powder was not dissolved in neutral solvent such as distilled water, and so the particle size of the obtained mixed powder could not be determined. However, the state of the final mixed powder was very similar to those of Example 4.

### INDUSTRIAL APPLICABILITY

According to the present invention, the loss of active ingredients and regrowth of particles can be prevented significantly, and thus finer nanoparticles of active ingredients can be prepared with good efficiency. Also, the loss of activity by the heat during the procedure for preparing nanoparticles can be reduced. The nanoparticles prepared by the present invention may be suitably used in medicinal products, functional or general foods, cosmetics and the like, due to their excellent dispersability, absorbing property, physiological activity and the like.

## Claims

1. A method for preparing nanoscale particles of active ingredient, comprising the steps of:
(1) preparing a mixture comprising one or more active ingredients and solid solvent,
(2) pressurizing the mixture comprising one or more active ingredients and solid solvent to 41 to 91 bar (40 to 90 atmosphere) at 10 to 20 °C by adding the gas of a supercritical fluid into a reaction vessel containing the mixture, and
(3) removing the solid solvent from the mixture by releasing out the solid solvent together with the gas of the supercritical fluid, with maintaining the temperature and pressure in the reaction vessel at 10 to 20 °C and 41 to 91 bar (40 to 90 atmosphere).

2. The method according to claim 1, wherein the active ingredient is one or more physiologically active materials selected from the group consisting of organic compounds, organometallic compounds, natural extracts, peptides, proteins and polysaccharides.

3. The method according to claim 1, wherein the solid solvent is selected from the group consisting of saturated fatty acids, esters and alcohols with C10-C22; mono- or di-glycerides having saturated fatty acid group with C10-C22; hydrocarbons with C16 or more; compounds having reduced fatty acid of tri-glycerides with C10~C22; linear or branched diol compounds with C6-C22; and mixtures thereof.

4. The method according to claim 1, wherein the solid solvent is diol compound.

5. The method according to claim 1, wherein the solid solvent is a mixture of diol compound and other solid solvent than diol.

6. The method according to claim 1, wherein the mixture comprising one or more active ingredients and solid solvent in the step (1) further comprises one or more surfactants.

7. The method according to claim 6, wherein the surfactant is one or more selected from the group consisting of synthetic surfactants, natural surfactants, lipids and polymers.

8. The method according to claim 1, wherein the mixture comprising one or more active ingredients and solid solvent in the step (1) further comprises one or more non-surfactant type anti-coagulating agents.

9. The method according to claim 8, wherein the non-surfactant type anticoagulating agent is one or more selected from the group consisting of monosaccharides, polysaccharides, dietary fibers, gums and proteins.

10. The method according to claim 1, wherein a co-solvent is further used in the step (1).

11. The method according to claim 10, wherein the co-solvent is one or more selected from the group consisting of alcohols, water, and mixtures thereof.

12. The method according to claim 11, wherein the co-solvent is one or more alcohols with C2-C6.

13. The method according to claim 11, wherein the co-solvent is one or more mixture solutions of 70-80 wt% of alcohol and 20-30 wt% of water.

14. The method according to any one of claims 1 to 13, wherein the pressure inside the reaction vessel in the step (3) is 51-81 bar (50~80 atm).

## Patentansprüche

1. Methode zur Herstellung von Nanopartikeln aus einem aktiven Inhaltsstoff, umfassend die Schritte:
(1) das Herstellen einer Mischung, die einen oder mehrere aktive Inhaltsstoffe und ein festes Lösungsmittel umfasst,
(2) das unter Druck Setzen der Mischung, die einen oder mehrere aktive Inhaltsstoffe und ein festes Lösungsmittel umfasst, von 41 bis 91 bar (40 bis 90 Atmosphären) bei 10 bis 20 °C durch das Hinzufügen des Gases von einer superkritischen Flüssigkeit in ein Reaktionsgefäß, das die Mischung enthält, und
(3) das Entfernen des festen Lösungsmittels von der Mischung durch Freilassen des festen Lösungsmittels zusammen mit dem Gas von der superkritischen Flüssigkeit, unter Erhalt der Temperatur und des Druckes in dem Reaktionsgefäß bei 10 bis 20 °C und 41 bis 91 bar (40 bis 90 Atmosphären).

2. Methode gemäß Anspruch 1, worin der aktive Inhaltsstoff ein oder mehrere physiologisch aktive Materialien ist, ausgewählt aus der Gruppe ist, die aus organischen Verbindungen, organometallischen Verbindungen, natürlichen Extrakten, Peptiden, Proteinen und Polysacchariden besteht.

3. Methode gemäß Anspruch 1, worin das feste Lösungsmittel aus der Gruppe ausgewählt ist, die aus gesättigten Fettsäuren, Estern und Alkoholen mit C10~C22; Mono- oder Diacylglycerinen, welche gesättigte Fettsäuregruppen mit C10~C22 haben; Kohlenwasserstoffen mit C16 oder mehr; Verbindungen, die reduzierte Fettsäuren von Triacylglycerinen mit C10~C20 haben; linearen oder verzweigten Diolverbindungen mit C6~C22; und Mischungen daraus besteht.

4. Methode gemäß Anspruch 1, worin das feste Lösungsmittel eine Diolverbindung ist.

5. Methode gemäß Anspruch 1, worin das feste Lösungsmittel eine Mischung aus einer Diolverbindung und festem Lösungsmittel unterschiedlich von Diol ist.

6. Methode gemäß Anspruch 1, worin die Mischung, die einen oder mehrere aktive Inhaltsstoffe und ein festes Lösungsmittel umfasst, in Schritt (1) weiterhin eine oder mehrere grenzflächenaktive Substanzen umfasst.

7. Methode gemäß Anspruch 6, worin die grenzflächenaktive Substanz eine oder mehrere aus der Gruppe ausgewählt ist, die aus synthetischen grenzflächenaktiven Substanzen, natürlichen grenzflächenaktiven Substanzen, Lipiden und Polymeren besteht.

8. Methode gemäß Anspruch 1, worin die Mischung, die einen oder mehrere aktive Inhaltsstoffe und ein festes Lösungsmittel umfasst, in dem Schritt (1) ferner eines oder mehrere Antikoagulationsmittel umfasst, welche nicht vom grenzflächenaktiven Substanztyp sind.

9. Methode gemäß Anspruch 8, worin das Antikoagulationsmittel, welches nicht vom grenzflächenaktiven Substanztyp ist, eines oder mehrere aus der Gruppe ausgewählt ist, die aus Monosacchariden, Polysacchariden, Ballaststoffen, Gummis und Proteinen besteht.

10. Methode gemäß Anspruch 1, worin ferner ein Zusatzlösungsmittel in Schritt (1) verwendet wird.

11. Methode gemäß Anspruch 10, worin das Zusatzlösungsmittel eines oder mehrere aus der Gruppe ausgewählt ist, die aus Alkoholen, Wasser und Mischungen daraus besteht.

12. Methode gemäß Anspruch 11, worin das Zusatzlösungsmittel ein oder mehrere Alkohole mit C2~C6 ist.

13. Methode gemäß Anspruch 11, worin das Zusatzlösungsmittel eine oder mehrere Mischungslösungen aus 70-80 Gew.-% Alkohol und 20-30 Gew.-% Wasser ist.

14. Methode gemäß einem der Ansprüche 1 bis 13, worin der Druck in dem Reaktionsgefäß in Schritt (3) 51-81 bar (50~80 atm) ist.

## Revendications

1. Procédé de préparation de nanoparticules de substance active, comprenant les étapes consistant à :
(1) préparer un mélange comprenant une ou plusieurs substances actives et un solvant solide,
(2) pressuriser le mélange comprenant une ou plusieurs substances actives et un solvant solide à une pression de 41 à 91 bar (de 40 à 90 atm) et à une température de 10 à 20 °C en ajoutant le gaz d'un fluide supercritique dans un récipient à réaction contenant le mélange, et
(3) extraire le solvant solide du mélange en libérant le solvant solide conjointement avec le gaz du fluide supercritique, en maintenant la température et la pression dans le récipient à réaction à une valeur de 10 à 20 °C et de 41 à 91 bar (de 40 à 90 atm).

2. Procédé selon la revendication 1, dans lequel la substance active est constituée d'une ou plusieurs matières physiologiquement actives choisies dans le groupe constitué des composés organiques, des composés organométalliques, des extraits naturels, des peptides, des protéines et des polysaccharides.

3. Procédé selon la revendication 1, dans lequel le solvant solide est choisi dans le groupe constitué des acides gras saturés, des esters et des alcools avec C10~C22 ; des mono- ou di-glycérides comportant un groupe acide gras saturé avec C10~C22 ; des hydrocarbures avec C16 ou plus ; des composés comportant un acide gras réduit de triglycérides avec C10~C22 ; des composés de type diols linéaires ou ramifiés avec C6~C22 ; et de leurs mélanges.

4. Procédé selon la revendication 1, dans lequel le solvant solide est un composé de type diol.

5. Procédé selon la revendication 1, dans lequel le solvant solide est un mélange de composé de type diol et d'un autre solvant solide autre que le diol.

6. Procédé selon la revendication 1, dans lequel le mélange comprenant une ou plusieurs substances actives et un solvant solide dans l'étape (1) comprend en outre un ou plusieurs tensioactifs.

7. Procédé selon la revendication 6, dans lequel le tensioactif est un ou plusieurs choisis dans le groupe constitué des tensioactifs synthétiques, des tensioactifs naturels, des lipides et des polymères.

8. Procédé selon la revendication 1, dans lequel le mélange comprenant une ou plusieurs substances actives et un solvant solide dans l'étape (1) comprend en outre un ou plusieurs agents anti-coagulants de type non-tensioactif.

9. Procédé selon la revendication 8, dans lequel l'agent anti-coagulant de type non-tensioactif est un ou plusieurs choisis dans le groupe constitué des monosaccharides, des polysaccharides, des fibres alimentaires, des gommes et des protéines.

10. Procédé selon la revendication 1, dans lequel un co-solvant est par ailleurs utilisé dans l'étape (1).

11. Procédé selon la revendication 10, dans lequel le co-solvant est un ou plusieurs choisis dans le groupe constitué des alcools, de l'eau et de leurs mélanges.

12. Procédé selon la revendication 11, dans lequel le co-solvant est un ou plusieurs alcools avec C2-C6.

13. Procédé selon la revendication 11, dans lequel le co-solvant est une ou plusieurs solutions mixtes de 70 à 80 % en poids d'alcool et de 20 à 30 % en poids d'eau.

14. Procédé selon l'une quelconque des revendications 1 à 13, dans lequel la pression à l'intérieur du récipient à réaction dans l'étape (3) est de 51 à 81 bar (de 50 à 80 atm).
